# EUROPÄISCHE PATENTANMELDUNG

(11) **EP 4 461 199 A2**
(43) Veröffentlichungstag der Anmeldung: **13.11.2024**
(21) Anmeldenummer: 24203847.9
(22) Anmeldetag: 14.01.2021
(51) Int. Cl.: A61B 1/00

(54) **TRIEBKÖRPER FÜR EINEN ALBARRAN**

(30) Priorität: 06.02.2020 DE 102020103016
(62) Teilanmeldung aus: 21151701.6
(71) Anmelder: Olympus Winter & Ibe GmbH, 22045 Hamburg (DE)
(72) Erfinder: Schulz, Kevin Alexander, 22889 Wilstedt (DE)
(74) Vertreter: Hoener, Matthias

(57) **Zusammenfassung**

Die Erfindung schafft einen Triebkörper (17) mit dem mindestens ein Zugdraht (21) auf eine besonders einfache und zuverlässige Art und Weise verspannbar ist. Das wird dadurch erreicht, dass ein Spannmittel zum Verspannen des mindestens einen Zugdrahtes (21) parallel zu einer Längsachse eines Schafts (12) mit einem Triebkörper (17) des Albarrans (10) verbindbar ist.

## Beschreibung

Die Erfindung betrifft einen Triebkörper gemäß dem Anspruch 1.

Albarrane werden unterstützend eingesetzt für Operationen bzw. Behandlungen mit chirurgischen Instrumenten wie beispielsweise Endoskopen, Resektoskopen, Zystoskopen oder dergleichen. Ein Albarran kann beispielsweise dazu verwendet werden, eine flexible Zange innerhalb eines Patienten auf eine gezielte sowie kontrollierte Art und Weise abzuwinkeln. Dazu weist der Albarran, genau wie beispielsweise ein Endoskop, einen stab- bzw. rohrartigen Schaft auf, welcher mit einem distalen Ende in den Körper des Patienten zu führen ist. An einem proximalen Ende des Albarrans, außerhalb des Patienten, ist der Schaft mit einem Hauptkörper verbunden. Über diesen Hauptkörper können weitere Instrumente bzw. Werkzeuge wie beispielsweise eine Optik, Drähte oder dergleichen über verschiedene Öffnungen bzw. Ports durch den Schaft in den Patienten geführt werden.

An dem distalen Ende des Schaftes weist der Albarran einen Hebel, den sogenannten Albarran-Hebel, auf. Dieser Hebel ist beweglich ausgestaltet und kann über einen Knebel am Hauptkörper betätigt bzw. verschwenkt werden. Dazu ist der Hebel mit dem Knebel entlang des Schaftes mechanisch gekoppelt. Bei dieser mechanischen Kopplung kann es sich entweder um einen Stab oder einen Zugdraht handeln. In der Regel ist der Hebel über zwei Zugdrähte mit dem Knebel verbunden. Es ist denkbar, dass beide Zugdrähte gleichermaßen dem Vor- und Zurückverschwenken des Hebels dienen oder über die beiden Zugdrähte verschiedene Bewegungen des Hebels über den Knebel bewirkbar sind.

Für eine zuverlässige Bedienung des Albarran-Hebels sind die Zugdrähte innerhalb des Hauptkörpers und dort in einem Triebkörper zu verspannen. Das heißt, dass die Zugdrähte während der Montage unter mechanischer Spannung verschraubt werden. Dazu werden die Zugdrähte bei bekannten Albarranen jeweils von einer Madenschraube gespannt. Damit die Madenschrauben den jeweiligen Zugdraht verspannen können, muss der Draht mittels Glühen verformbar gemacht werden. Dieser Schritt gilt als besonders umständlich, da der bereits in dem Instrument verlegte Draht in dem Hauptkörper mit einer ausreichend großen Wärmeenergie zu beaufschlagen und sodann mit der Madenschraube zu verspannen ist. Da diese Verspannung von der Seite erfolgen muss, d. h. quer zu einer Längsachse des Schaftes, muss dieser Montageschritt vor der Installation des Knebels bzw. einer Knebelachse erfolgen. Die Madenschrauben werden über die freien Öffnungen für die Knebel in dem Triebkörper verschraubt. Insgesamt gestaltet sich diese Montage als sehr aufwendig, kompliziert und somit als fehleranfällig. Sofern der Hauptkörper des Albarran einen derartigen seitlichen Zugang zu dem Zugdraht nicht zulässt, kann diese Art der Verspannung nicht erfolgen.

Der Erfindung liegt die Aufgabe zugrunde einen Triebkörper für einen Albarran zu schaffen, mit dem der Zugdraht auf eine besonders einfache und zuverlässige Art und Weise verspannbar ist.

Eine Lösung dieser Aufgabe wird durch die Merkmale des Anspruchs 1 beschrieben. Demnach ist es vorgesehen, dass das Spannmittel zum Verspannen des mindestens einen Zugdrahtes parallel zu einer Längsachse des Schafts mit dem Triebkörper verbindbar ist. Durch diese parallele Verspannung des Zugdrahtes ist kein seitlicher Zugang zu dem Zugdraht mehr erforderlich. Vielmehr kann der Zugdraht bzw. können die Zugdrähte vom proximalen Ende des Triebkörpers her auf eine einfache Art und Weise verspannt werden. In der Regel weist der Triebkörper an seinem proximalen Ende eine Kappe auf, die auf eine einfache Art und Weise von dem Triebkörper gelöst werden kann. Durch diese Verspannung des Zugdrahtes parallel zur Längsachse des Schaftes kann eine besonders einfache sowie zuverlässige Verspannung erfolgen.

Bevorzugt kann es erfindungsgemäß weiter vorgesehen sein, dass es sich bei dem Spannmittel um eine Schraube handelt, die parallel zu dem Schaft im Triebkörper bzw. mit einem Zugdrahtmitnehmer im Triebkörper verschraubbar ist, wobei die Schraube von einer proximalen Seite des Triebkörpers, vorzugsweise von einer geöffneten proximalen Seite des Triebkörpers aus, in dem Triebkörper verschraubbar ist. Durch diese Verschraubung des Zugdrahtes bzw. der Zugdrähte, vom proximalen Ende her entlang der Schaftachse, wird eine Erhitzung des Drahtes für die Verspannung überflüssig. Dadurch vereinfacht sich die gesamte Montage des Albarrans.

Insbesondere kann es die Erfindung weiter vorsehen, dass ein Schraubenkopf der Schraube mit einem Deckel bewegungsgekoppelt ist, wobei durch das Anziehen der Schraube der Deckel in den Triebkörper bzw. in den Zugdrahtmitnehmer gezogen wird und zwischen dem Deckel und dem Zugdrahtmitnehmer der mindestens eine Zugdraht verspannbar ist. Über die Schraube lässt sich somit eine definierte Kraft für die Verspannung des Drahtes zwischen dem Deckel und dem Zugdrahtmitnehmer aufwenden. Diese definierte Vorspannung gewährt eine hohe Verlässlichkeit der mechanischen Verbindung zu dem Albarran-Hebel.

Weiter kann es erfindungsgemäß vorgesehen sein, dass der Deckel einen trapezartigen Querschnitt aufweist bzw. pyramidenstumpfartig ausgebildet ist, wobei der trapezartige Querschnitt bzw. der Pyramidenstumpf in distaler Richtung des Schaftes konvergiert und der Triebkörper bzw. der Zugdrahtmitnehmer eine korrespondierende Aufnahme zur Aufnahme des Deckels aufweist. Der Querschnitt des Deckels kann insbesondere ein gleichschenkeliges oder ein beliebigschenkeliges Trapez beschreiben. Durch diese miteinander korrespondierende Formgebung des Deckels und des Zugdrahtmitnehmers ist eine besonders effiziente und sichere Verspannung des Drahtes möglich. Durch Festziehen der Schraube wird der Deckel automatisch in die korrespondierend ausgebildete Aufnahme des Zugdrahtmitnehmers gezogen. Bei diesem Prozess der Verspannung ist der Draht bzw. sind die Drähte auf Zug zu halten, um die gewünschte mechanische Spannung zwischen dem Knebel und dem Hebel zu erreichen. Durch den in Schaftrichtung betrachtet polygonalen, vorzugsweise rechteckigen, Querschnitt des Deckels kann sichergestellt werden, dass sich der Deckel beim Festziehen der Schraube in dem Zugdrahtmitnehmer nicht mitdreht; vielmehr verkantet der Deckel mit seinen Ecken in der Aufnahme des Zugdrahtmitnehmers. Um sich der Form des Triebkörpers bzw. des Zugdrahtmitnehmers besser anzupassen bzw. den zur Verfügung stehenden Platz optimal zu nutzen, ist es denkbar, dass wenigstens eine Seite des Deckels konvex ausgebildet ist, wobei die konvexe Form mit der Form des Triebkörpers korrespondieren kann.

Ein weiteres Ausführungsbeispiel der Erfindung kann es vorsehen, dass eine Umfangsfläche des Deckels strukturiert bzw. angeraut ist, um den Reibwiderstand zwischen der Umfangsfläche und dem Draht und der Aufnahme zum Verspannen zu erhöhen. Die Aufrauung kann beispielsweise auch durch Strahlen erfolgen. Durch diese Rauigkeit wird vermieden, dass bei einer erhöhten Krafteinwirkung auf den Albarran-Hebel die Zugdrähte aus dem Triebkörper herausgezogen werden.

Ein besonders vorteilhaftes Ausführungsbeispiel der Erfindung kann es vorsehen, dass der mindestens eine Zugdraht und die Schraube zum Verspannen des Drahtes in den Triebkörper unterhalb einer quer zur Längsachse des Schaftes ausgerichteten Achse des Knebels angeordnet sind. Durch diese Geometrie bzw. Positionierung des Verspannungsmittels bietet der Hauptkörper Platz für weitere Ports bzw. Öffnungen in dem Hauptkörper zur Einführung weiterer Werkzeuge oder Instrumente.

Bevorzugterweise kann es außerdem vorgesehen sein, dass zum Verspannen des mindestens einen Zugdrahts ein distales Ende des Drahtes durch den Triebkörper bzw. durch mindestens eine Öffnung im Zugdrahtmitnehmer ziehbar ist und durch Verschrauben des Deckels das Drahtende zwischen dem Deckel und der Aufnahme verspannbar ist.

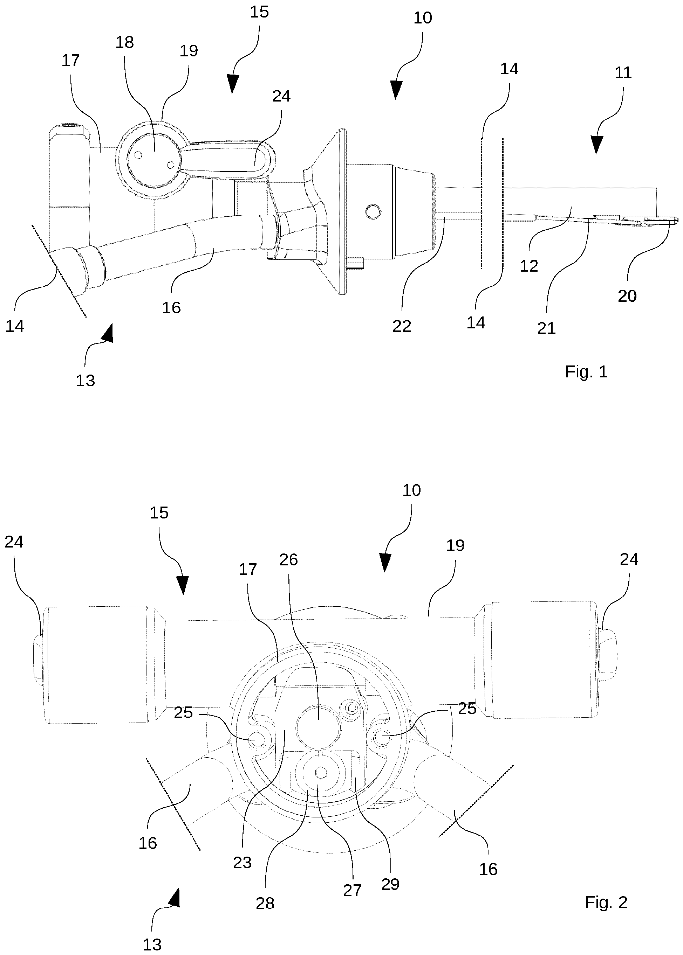

Ein bevorzugtes Ausführungsbeispiel der vorliegenden Erfindung wird nachfolgend anhand der Zeichnung näher erläutert. In dieser zeigen:
- .Fig. 1.: eine schematische Darstellung eines Albarrans, und
- Fig. 2: eine Sicht auf ein proximales Ende des Albarrans gemäß der Fig. 1, und
- Fig. 3: eine perspektivische Darstellung eines Deckels.

In der Fig. 1 ist ein mögliches Ausführungsbeispiel eines Albarrans 10 dargestellt. Der Anschauung halber ist lediglich ein distales Ende 11 eines Schaftes 12 sowie ein proximales Ende 13 des Albarrans 10 dargestellt. Durch die angedeuteten Schnittlinien 14 ist der langgestreckte, rohrartige Schaft 12 nur teilweise wiedergegeben. Da der nicht dargestellte Abschnitt des Schaftes 12 keine erfindungswesentlichen Merkmale aufweist, genügt diese reduzierte Darstellung dem Verständnis der Erfindung.

Das proximale Ende 13 des Albarrans 10 wird durch einen Hauptkörper 15 gebildet. Dieser Hauptkörper 15 ist direkt mit dem Schaft 12 verbunden. Während für eine Behandlung bzw. Operation eines Patienten der längliche Schaft 12 in ein Körperinneres des Patienten geführt wird, bleibt der Hauptkörper 15 außerhalb des Körpers und dient der Bereitstellung verschiedener Werkzeuge bzw. Hilfsmittel zur Durchführung der Operation. Dazu sind dem Hauptkörper 15 beispielsweise zwei rohrartige Ports 16 zugeordnet, durch welche nicht dargestellte Werkzeuge oder andere Hilfsmittel während der Operation durch den Schaft 12 zum distalen Ende 11 des Albarrans 10 zum Ort der Operation führbar sind. Auch hier sind der Anschauung halber die proximalen Enden der Ports 16 bzw. beispielhaft folgende Ventile nicht dargestellt. Auch diese stellen, wie oben für den Schaft beschrieben, Merkmale dar, die für die Beschreibung der Erfindung unwesentlich sind.

Des Weiteren weist der Hauptkörper 15 einen Triebkörper 17 auf. Durch diesen Triebkörper 17 führt senkrecht zu einer Längsachse des Albarrans 10 bzw. des Schaftes 12 eine Knebelachse 18 eines Knebels 19. Durch die Betätigung dieses hebelartigen Knebels 19 bzw. durch Rotation des Knebels 19 um die Knebelachse 18, lässt sich ein Albarran-Hebel 20 am distalen Ende 11 des Albarrans 10 betätigen. Dieser Albarran-Hebel 20 dient während der Operation bzw. der Behandlung als Hilfswerkzeug. Durch Betätigung dieses Hebels 20 können weitere nicht dargestellte Werkzeuge, die durch andere chirurgische Instrumente, wie beispielsweise Endoskope oder Zystoskope in den Körper hineingeführt wurden, unterstützend bedient werden. Durch ein Abwinkeln des Albarran-Hebels 20 im Uhrzeigersinn lässt sich beispielsweise eine flexible Zange im Körperinneren bewegen.

Für die Betätigung des Albarran-Hebels 20 ist der Triebkörper 17 bzw. der Knebel 19 mit einem bzw. zwei Zugdrähten 21 bewegungsgekoppelt. Dieser Zugdraht 21 kann entweder innerhalb des Schaftes 12 geführt werden oder außerhalb des Schaftes 12 durch ein Röhrchen 22. Dieses Röhrchen 22 verläuft parallel zu dem Schaft 12 von dem proximalen Ende 13 zum distalen Ende 11 bzw. zum Albarran-Hebel 20. Dort ist der Zugdraht 21 mit dem Albarran-Hebel 20 derart gekoppelt, dass eine Betätigung des Albarran-Hebels 20 möglich ist. An einem proximalen Ende des Zugdrahtes 21 ist dieser im Triebkörper 17 bzw. mit einem Zugdrahtmitnehmer 23 verbunden. Der Zugdraht 21 ist zwischen dem Zugdrahtmitnehmer 23 und dem Albarran-Hebel 20 verspannt, das heißt, dass der mindestens eine Zugdraht 21 unter mechanischer Spannung zwischen dem Zugdrahtmitnehmer 23 und dem Albarran-Hebel 20 innerhalb des Hauptkörpers 15 bzw. des Röhrchens 22 oder des Schaftes 12 montiert ist. Durch Betätigung des Knebels 19 um die Knebelachse 18 wird der mindestens eine Zugdraht 21 weiter gespannt, sodass sich der Albarran-Hebel 20 um eine Schwenkachse dreht. Sobald der Knebel 19 in eine entgegengesetzte Richtung gedreht wird, lässt die mechanische Spannung auf den Zugdraht 21 nach und der Albarran-Hebel 20 bewegt sich zurück in seine Ausgangsposition. Für die Betätigung des Knebels 19 weist dieser, wie in Fig. 2 sichtbar, zwei Betätigungsmittel 24 auf. Diese hebelartigen Betätigungsmittel 24 sind an dem Knebel 19 beiden Seiten des Hauptkörpers 15 zugeordnet.

Die Fig. 2 gewährt eine Sicht auf ein offenes proximales Ende des Hauptkörpers 15 bzw. des Triebkörpers 17. Eine nicht dargestellte Kappe lässt sich auf dieses offene Ende des Hauptkörpers 15 setzen und mittels Schrauben und den Gewindebohrungen 25 verschließen. Zentral in dem Triebkörper 17 ist ein Kanal 26 angeordnet, der sich durch den gesamten Albarran 10 und durch den Schaft 12 bis zum distalen Ende 11 erstreckt. Durch diesen Kanal 26 kann beispielsweise für die Durchführung einer Operation eine Optik geführt werden. Diese nicht dargestellte Optik dient beispielsweise der Beobachtung der Operation oder einem gezielten Ansteuern des Albarran-Hebels 20.

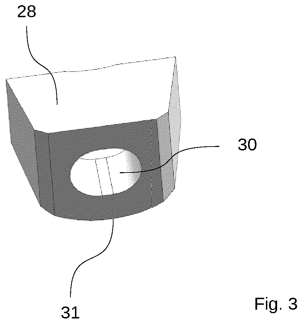

Unterhalb des Kanals 26 und unterhalb der Knebelachse 18 weist der Zugdrahtmitnehmer 23 eine Schraube 27, insbesondere eine Senkkopfschraube, auf. Diese Schraube 27 ist in den Triebkörper 17 geschraubt und zwar parallel zur Längsachse des Schaftes 12. Mit der Schraube 27 ist ein Deckel 28 (Fig. 3) in eine Aufnahme 29 des Zugdrahtmitnehmers 23 gefügt bzw. arretiert. Dazu wird die Schraube 27 durch eine Bohrung 30 durch den Deckel 28 geführt. Dieser Deckel 28 kann konisch ausgebildet sein, wobei sein Umfang in Richtung zum distalen Ende 11 hin konvergiert. Es ist allerdings auch denkbar, dass der Deckel 28 einen trapezartigen Querschnitt aufweist bzw. pyramidenstumpfartig ausgebildet ist. Der trapezartige Querschnitt bzw. der Pyramidenstumpf des Deckels 28 konvergiert dabei ebenfalls in distale Richtung des Schaftes (Fig. 3). Der Triebkörper 17 bzw. der Zugdrahtmitnehmer 23 weisen für einen Formschluss eine zu der Form des Deckels 28 korrespondierende Aufnahme 29 auf. Durch diese miteinander korrespondierende Formgebung des Deckels 28 und des Zugdrahtmitnehmers 23 ist eine besonders effiziente und sichere, insbesondere formschlüssige, Verspannung des Drahtes möglich. Durch Festziehen der Schraube 27 wird der Deckel 28 automatisch in die korrespondierend ausgebildete Aufnahme 29 des Zugdrahtmitnehmers 23 gezogen. Der polygonale, vorzugsweise rechteckige, Querschnitt des Deckels 28 stellt sicher, dass der Deckel 28 beim Festziehen der Schraube 27 nicht mitdreht. Durch die Form verkantet der Deckel 28 in der Aufnahme 29 des Zugdrahtmitnehmers 23. Das in der Fig. 3 beispielhaft dargestellte Ausführungsbeispiel des Deckels 28 weist eine konvexe Seitenwandung 31 auf. Durch diese Formgebung der Seitenwandung 31 kann sich der Deckel 28 der Form des Triebkörpers 17 bzw. des Zugdrahtmitnehmers 23 besser anpassen.

Bei dem hier dargestellten Ausführungsbeispiel des Albarrans 10 wird zur Montage des Zugdrahtes 21 selbiger vom distalen Ende 11 des Albarrans 10 durch das Röhrchen 22 und den Hauptkörper 15 bzw. den Triebkörper 17 gezogen. Die proximalen Enden des Zugdrahtes 21 werden dann durch eine bzw. zwei Öffnungen in dem Zugdrahtmitnehmer 23 parallel zu der Gewindebohrung der Schraube 27 gezogen. Während die beiden Zugdrähte 21 unter mechanischer Spannung gehalten werden, wird die Schraube 27 mitsamt des Deckels 28 in die Aufnahme 29 verschraubt. Dadurch werden die Zugdrähte 21 zwischen der Aufnahme 29 und dem Umfang des Deckels 28 verspannt. Diese Verspannung lässt sich besonders einfach durchführen, da keine weiteren Werkzeuge oder Verfahrensschritte notwendig sind. Durch die gezielte Ausübung eines definierten Drehmomentes auf die Schraube 27 können die Zugdrähte 21 mit einer hohen Genauigkeit und Verlässlichkeit verspannt werden. Nach dem Abtrennen der freien Enden der Zugdrähte 21 kann die Kappe auf den Hauptkörper 15 verschraubt werden. Um die auf die Zugdrähte 21 wirkende Spannkraft zu erhöhen, kann der Umfang des Deckels 28 angeraut werden. Durch diese Anrauung wird eine Reibkraft zwischen der Aufnahme 29, dem Deckel 28 sowie den Drähten 21 erzeugt, die zu einer höheren Verspannkraft führt.

Es sei ausdrücklich darauf hingewiesen, dass die hier beschriebenen Merkmale nicht auf die Anwendung des dargestellten Ausführungsbeispiels eines Albarrans 10 eingeschränkt sind. Vielmehr ist es denkbar, dass die erfindungsgemäßen Merkmale auch im Zusammenhang mit anderen chirurgischen Instrumenten Anwendung finden können.

### Bezugszeichenliste:

- 10: Albarran
- 11: distales Ende
- 12: Schaft
- 13: proximales Ende
- 14: Schnittlinie
- 15: Hauptkörper
- 16: Port
- 17: Triebkörper
- 18: Knebelachse
- 19: Knebel
- 20: Albarran-Hebel
- 21: Zugdraht
- 22: Röhrchen
- 23: Zugdrahtmitnehmer
- 24: Betätigungsmittel
- 25: Gewindebohrung
- 26: Kanal
- 27: Schraube
- 28: Deckel
- 29: Aufnahme
- 30: Bohrung
- 31: Seitenwandung

## Patentansprüche

1. Triebkörper (17) für einen Albarran (10), wobei der Albarran (10) einen Schaft (12) aufweist, an dessem distalen Ende (11) ein Albarran-Hebel (20) und an dessem proximalen Ende (13) ein Hauptkörper (15) des Triebkörpers (17) angeordnet ist, wobei der Albarran-Hebel (20) über mindestens einen Zugdraht (21) durch einen Knebel (19) am Triebkörper (17) bewegbar und ein proximales Ende des mindestens einen Zugdrahts (21) im Triebkörper (17) durch ein Spannmittel verspannt ist, **dadurch gekennzeichnet, dass** das Spannmittel zum Verspannen des mindestens einen Zugdrahts (21) parallel zu einer Längsachse des Schaftes (12) mit dem Triebkörper (17) verbindbar ist.

2. Triebkörper (17) für einen Albarran (10) nach Anspruch 1, **dadurch gekennzeichnet, dass** es sich bei dem Spannmittel um eine Schraube (27) handelt, die parallel zu dem Schaft (12) im Triebkörper (17) bzw. mit einem Zugdrahtmitnehmer (23) im Triebkörper (17) verschraubbar ist, wobei die Schraube (27) von einer proximalen Seite (13) des Triebköpers (17), vorzugsweise von einer geöffneten proximalen Seite des Triebkörpers (17) aus, in den Triebkörper (17) verschraubbar ist.

3. Triebkörper (17) für einen Albarran (10) nach Anspruch 2, **dadurch gekennzeichnet, dass** ein Schraubenkopf der Schraube (27) mit einem Deckel (28) bewegungsgekoppelt ist, wobei durch das Anziehen der Schraube (27) der Deckel (28) in den Triebkörper (17) bzw. in den Zugdrahtmitnehmer (23) gezogen wird und zwischen dem Deckel (28) und dem Zugdrahtmitnehmer (23) der mindestens eine Zugdraht (21) verspannbar ist.

4. Triebkörper (17) für einen Albarran (10) nach Anspruch 3, **dadurch gekennzeichnet, dass** der Deckel (28) einen trapezartigen Querschnitt aufweist bzw. pyramidenstumpfartig ausgebildet ist, wobei der trapezartige Querschnitt bzw. der Pyramidenstumpf in distale Richtung des Schaftes (12) konvergiert und der Triebkörper (17) bzw. der Zugdrahtmitnehmer (23) eine korrespondierende Aufnahme (29) zur Aufnahme des Deckels (28) aufweist.

5. Triebkörper (17) für einen Albarran (10) nach Anspruch 3 oder 4, **dadurch gekennzeichnet, dass** eine Umfangsfläche des Deckels (28) strukturiert bzw. angeraut ist, um den Reibwiderstand zwischen der Umfangsfläche, dem Draht (21) und der Aufnahme (29) zum Verspannen zu erhöhen.

6. Triebkörper (17) für einen Albarran (10) nach Anspruch 2, **dadurch gekennzeichnet, dass** der mindestens eine Zugdraht (21) und die Schraube (27) zum Verspannen des Drahtes (21) in dem Triebkörper (17) unterhalb einer quer zur Längsachse des Schaftes (12) ausgerichteten Achse des Knebels (19) angeordnet sind.

7. Triebkörper (17) für einen Albarran (10) nach Anspruch 3, **dadurch gekennzeichnet, dass** zum Verspannen des mindestens einen Zugdrahts (21) ein distales Ende des Drahtes (21) durch den Triebkörper (17) bzw. durch mindestens eine Öffnung im Zugdrahtmitnehmer (23) ziehbar ist und durch Verschrauben des Deckels (28) das Drahtende zwischen dem Deckel (28) und der Aufnahme (29) verspannbar ist.
